# EUROPEAN PATENT SPECIFICATION

(11) **EP 1 988 960 B1**
(45) Date of publication and mention of the grant of the patent: **09.12.2009**
(21) Application number: 07777711.8
(22) Date of filing: 01.02.2007
(51) Int. Cl.: A61M 29/00, A61B 17/22

(54) **SYSTEM AND METHOD FOR TREATING A VASCULAR CONDITION**
SYSTEM UND VERFAHREN ZUR BEHANDLUNG EINER GEFÄßERKRANKUNG
SYSTÈME ET MÉTHODE POUR TRAITER UNE MALADIE VASCULAIRE

(30) Priority: 10.02.2006 US 276023
(43) Date of publication of application: 12.11.2008
(73) Proprietor: Medtronic Vascular, Inc., Santa Rosa, CA 95403 (US)
(72) Inventor: BERGLUND, Joseph, Santa Rosa, California 95403 (US)
(74) Representative: Zimmermann & Partner
(86) International application number: PCT/US2007/061454
(87) International publication number: WO 2007/117755

(56) References cited:
- EP-A- 1 604 704
- WO-A-02/43796
- DE-A1-102004 026 089
- US-A- 5 653 690
- US-A1- 2003 050 662
- US-B1- 6 197 013

## Description

### TECHNICAL FIELD OF THE INVENTION

The present invention relates generally to the field of vascular therapies. More particularly, the invention relates to a system and method for treating a vascular condition.

### BACKGROUND OF THE INVENTION

Heart disease, specifically coronary artery disease (CAD), is a major cause of death, disability, and healthcare expense. Until recently, most heart disease was considered primarily the result of a progressive increase of hard plaque in the coronary arteries. This atherosclerotic disease process of hard plaques leads to a critical narrowing (stenosis) of the affected coronary artery and produces anginal syndromes, known commonly as chest pain. The progression of the narrowing reduces blood flow, triggering the formation of a blood clot. The clot may choke off the flow of oxygen rich blood (ischemia) to heart muscles, causing a heart attack. Alternatively, the clot may break off and lodge in another organ vessel such as the brain resulting in a thrombotic stroke.

Within the past decade or so, evidence has emerged expanding the paradigm of atherosclerosis, coronary artery disease, and heart attacks. While the build up of hard plaque may produce angina and severe ischemia in the coronary arteries, new clinical data now suggests that the rupture of sometimes non-occlusive, vulnerable plaques causes the vast majority of heart attacks. The rate is estimated as high as 60-80 percent. In many instances vulnerable plaques do not impinge on the vessel lumen, rather, much like an abscess they are ingrained under the arterial wall. For this reason, conventional angiography or fluoroscopy techniques are unlikely to detect the vulnerable plaque. Due to the difficulty associated with their detection and because angina is not typically produced, vulnerable plaques may be more dangerous than other plaques that cause pain.

The majority of vulnerable plaques include a lipid pool, necrotic smooth muscle (endothelial) cells, and a dense infiltrate of macrophages contained by a thin fibrous cap, some of which are only two micrometers thick or less. The lipid pool is believed to be formed as a result of pathological process involving low density lipoprotein (LDL), macrophages, and the inflammatory process. The macrophages oxidize the LDL producing foam cells. The macrophages, foam cells, and associated endothelial cells release various substances, such as tumor necrosis factor, tissue factor, and matrix proteinases. These substances can result in generalized cell necrosis and apoptosis, pro-coagulation, and weakening of the fibrous cap. The inflammation process may weaken the fibrous cap to the extent that sufficient mechanical stress, such as that produced by increased blood pressure, may result in rupture. The lipid core and other contents of the vulnerable plaque (emboli) may then spill into the blood stream thereby initiating a clotting cascade. The cascade produces a blood clot (thrombosis) that potentially results in a heart attack and/or stroke. The process is exacerbated due to the release of collagen and other plaque components (e.g., tissue factor), which enhance clotting upon their release.

Given the prevalence and serious sequelae of vulnerable plaque, strategies are continuously being developed for detection and treatment. An endovascular approach to vulnerable plaque detection and/or treatment provides numerous advantages over other forms of surgery, such as traditional open surgery. The endovascular approach is offered not only to the otherwise healthy patient, but also to the elderly patient, who because of other health issues, could not have vulnerable plaque repaired by other procedures (e.g., conventional open procedures). In addition, the endovascular approach limits the trauma and some risk to the patient. Many traditional forms of 'open' surgery may produce significant trauma to the patient because of the need to access and stabilize a surgical site. For example, conventional coronary artery bypass graft (CABG) surgery may involve a medial sternotomy and connection to a heart-lung machine so that the surgeon may work on an exposed and still heart. Because of the trauma, the patient may experience a prolonged recovery time, increased pain and complications, and an overall worsening in prognosis. As such, it may be advantageous to utilize an endovascular approach for the detection and treatment of vulnerable plaque.

Several endovascular strategies have been developed for the detection (e.g., diagnosis and localization) of vulnerable plaques. One strategy involves the measurement of temperature within a blood vessel. A localized increase in temperature is generally associated with the vulnerable plaque because of the tissue damage and inflammation. It has been observed that the inflamed necrotic core of the vulnerable plaque maintains a temperature of one or more degrees Celsius higher than that of the surrounding tissue. For example, a relatively normal vessel temperature may be about 37°C whereas the vulnerable plaque may have a localized temperature as high as 40°C. Measurement of these temperature differences within the blood vessel may provide means for locating vulnerable plaque. Additionally, numerous other physical properties, changes, factors, molecules, and the like specific to the vulnerable plaque have been used to facilitate vulnerable plaque detection using and endovascular device. For example, localized changes in pH and an elevated serum concentration of C-reactive protein have been associated with heart attack, stroke, and vulnerable plaque.

Another detection strategy involves labeling vulnerable plaque with a marker and subsequent detection with an endovascular device. The marker substance may be specific for a component and/or characteristic of the vulnerable plaque. For example, the marker may have an affinity for the vulnerable plaque, more so than for healthy tissue. Detection of the marker may thus allow detection of the vulnerable plaque. An intravenous solution containing a radioactive tracer, which specifically accumulates in the vulnerable plaque, is administered to the patient. A miniaturized radiation endovascular device is positioned within the patient's arterial lumen (e.g., endovascularly) for localized radioactivity imaging and detection. The radiation detector identifies and differentiates vulnerable plaque from inactive, stable plaque. Alternatively, the marker may not necessarily have an affinity for the vulnerable plaque, but will simply change properties while associated with the vulnerable plaque. The property change may be detected and thus allow detection of the vulnerable plaque.

A number of therapies have been proposed to treat vulnerable plaque. The therapies range from stent devices designed to reduce fibrous cap loads, to methods designated to puncture the plaque wall and extract the clot-inducing core material, to stent-like devices capable of non-invasive inductive heating. Other proposed treatments utilize directed ultrasonic energy, radiation sources, and electrical pulses; rupture devices in conjunction with embolic capture devices; delivery of stabilizing therapeutic agents via stable or bioresorbable implantable devices; matrix metalloproteinase inhibitors; cryogenesis; and coating the vascular wall with a substance to prevent rupture.

While many of these therapies exhibit promise in therms of their therapeutic value, a commercially available treatment for vulnerable plaque has yet to be developed. Furthermore, many of these treatments provide short-term therapy that occurs only while the intervention is taking place or a permanent device solution that remains in place long after the therapy is necessary. In the case of deliberate plaque rupture and embolic capture, it is unclear whether emboli and clotting will subside after the intervention has been completed or whether the plaque will redevelop. Since vulnerable plaques typically do not result in blood flow restriction, it would be desirable to provide a therapy to stabilize the fibrous cap while maintaining minimal risk of rupture and tissue damage.

WO 02/43796 describes an apparatus for delivery of biologically active materials comprising a catheter and balloon having micro-needles or pores. In the apparatus, the balloon can have a polymer coating containing the biologically active material, and the apparatus can include a sheath surrounding the balloon. In one example the biologically active material is delivered through lumens in the micro-needles.

EP 1 604 704 describes a device for delivering a therapeutic agent into tissue, the device comprising an elongated body; a first balloon in the body expandable from a collapsed position to an expanded position; a second balloon in the body expandable from a collapsed position to an expanded position, the second balloon having a plurality of apertures therein; a therapeutic agent in the second balloon; a plurality of microprojections on a surface of the second balloon for penetrating tissue; and wherein upon expansion of the first balloon from the collapsed position to the expanded position, the second balloon is expanded from the collapsed position to the expanded position for deploying the plurality of microprojections into tissue and for dispensing the therapeutic agent from the second balloon into the tissue through the plurality of apertures.

DE 10 2004 026089 describes a catheter comprising at the head of it, around the periphery, exit openings which are provided for one or more guide channels, where each channel accommodates a sliding injection needle, and the channels are located in various radial positions, with uniform distribution.

US 2003/050662 describes a device for treating conditions causing obstructions in a body passage. The device is composed of: a first catheter dimensioned to be insertable into the body passage and having a lateral wall, a proximal end and a distal end; and a first balloon carried by the first catheter and extending outwardly from the lateral wall. The first catheter is provided internally with not more than three fluid conducting passages, including: a blood bypass flow passage extending at least from a first point located between the first balloon and the proximal end to a second point at the distal end and communicating at the first point with a region surrounding the first catheter; a balloon inflation passage communicating with the first balloon; and a delivery/aspiration passage opening at the lateral wall at a location between the first point and the first balloon.

US 5,653,690 describes a catheter for retrograde perfusion of the heart through the coronary sinus, which has an infusion lumen for introducing perfusion liquid into the heart, a retention means such as an inflatable balloon, and can have retention enhancements such as proximally sloping spikes, or barbed protuberances on the surface of the retention means to keep it firmly in place.

Accordingly, it would be desirable to provide a system and method for treating a vascular condition that would overcome the aforementioned and other disadvantages.

### SUMMARY OF THE INVENTION

A first aspect according to the invention provides a system for treating a vascular condition. The system includes a catheter and an inflatable member operably attached to the catheter. A plurality of biodegradable and detachable microdarts is disposed on the inflatable member.

Such a system may be applied in a method for treating a vascular condition. The method includes locating a vulnerable plaque. An inflatable member including a plurality of biodegradable and detachable microdarts is positioned adjacent the vulnerable plaque. The plurality of biodegradable microdarts is inserted into a vessel wall.

A third aspect according to the invention provides a system for treating a vascular condition. The system includes means for locating a vulnerable plaque, means for positioning an inflatable member including a plurality of biodegradable and detachable microdarts adjacent the vulnerable plaque, and means for inserting the plurality of biodegradable microdarts into a vessel wall.

The foregoing and other features and advantages of the invention will become further apparent from the following detailed description of the presently preferred embodiments, read in conjunction with the accompanying drawings. The detailed description and drawings are merely illustrative of the invention, rather than limiting the scope of the invention being defined by the appended claims and equivalents thereof.

### BRIEF DESCRIPTION OF THE DRAWINGS

**FIG. 1** illustrates a system for treating a vascular condition in accordance with the present invention;
**FIG. 2** illustrates a microdart in accordance with the present invention;
**FIG. 3** illustrates a balloon and a distal protection device deployed within a vessel, in accordance with the present invention;
**FIG. 4** illustrates a flowchart of a method of treating a vascular condition;
**FIG. 5** illustrates a plurality of microdarts inserted in a vessel wall, in accordance with the present invention; and
**FIG. 6** illustrates a treated vessel, in accordance with the present invention.

### DESCRIPTION OF THE PRESENTLY PREFERRED EMBODIMENTS

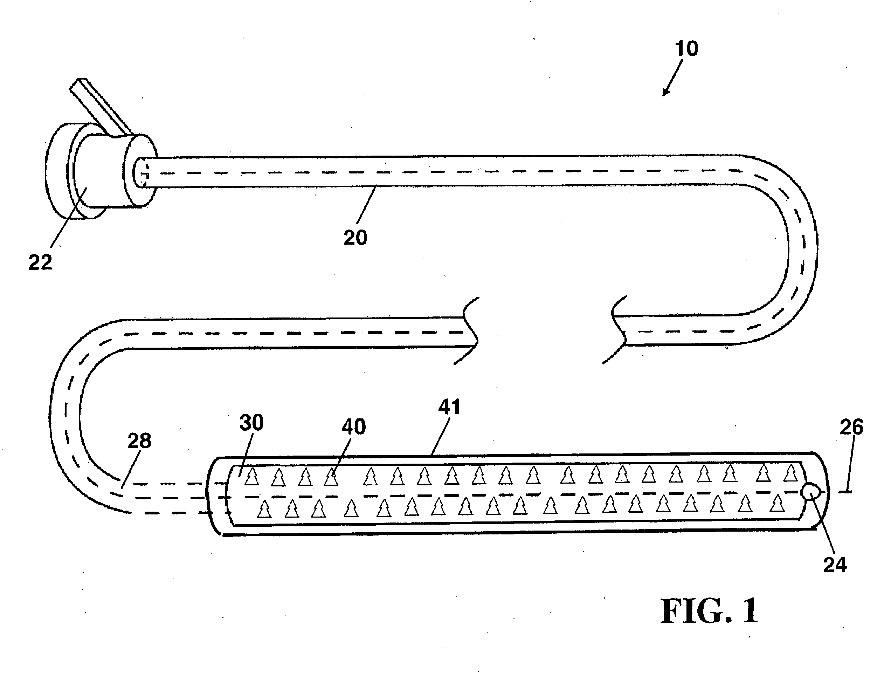

Referring to the drawings, which are not necessarily drawn to scale and wherein like reference numerals refer to like elements, **FIG. 1** is a perspective view of a system for treating a vascular condition in accordance with one embodiment of the present invention and shown generally by numeral **10.** System **10** includes a catheter **20,** a balloon **30** operably attached to the catheter **20,** and a plurality of biodegradable and detachable microdarts **40** disposed on the balloon **30.**

Balloon **30** is in a compressed configuration (as shown) during advancement through the vasculature maintaining a minimal profile size. In one embodiment, a sheath **41** is disposed over the balloon **30** to prevent contact between the microdarts **40** and the vessel walls. In another embodiment, a hydrogel coating (not shown) can be used to cover the microdarts **40** providing protection as well as lubrication during catheter **20** advancement. Those skilled in the art will recognize that numerous strategies can be employed for reducing contact of the vessel wall by the microdarts **40** or vice versa during catheter advancement. For example, the microdarts **40** can retract or fold into/alongside the balloon **30** in a manner that would allow them to be exposed only during the time of insertion.

Although the devices described herein are primarily done so in the context of treatment of a vulnerable plaque, it should be appreciated that other vascular conditions may benefit from the therapies disclosed herein. Further, the deployment of the microdarts is not limited to blood vessels, but can also include other vessels, such as a bile duct, intestinal tract, esophagus, and airway.

The term "biodegradable" refers to substances that degrade (i.e., via hydrolysis) to at least a certain extent within the body. Biodegradable substances are biocompatible and incur a reduced inflammatory response.

In one embodiment, catheter **20** includes an elongated tubular member manufactured from one or more polymeric materials, sometimes in combination with metallic reinforcement. In some applications (such as smaller, more tortuous arteries), it is desirable to construct the catheter from very flexible materials to facilitate advancement into intricate access locations. Numerous over-the-wire, rapid-exchange, and other catheter designs are known and may be adapted for use with the present invention. Catheter **20** can be secured at its proximal end to a suitable Luer fitting **22,** and can include a distal rounded end **24** to reduce harmful contact with a vessel. Catheter **20** can be manufactured from a material such as a thermoplastic elastomer, urethane, polymer, polypropylene, plastic, ethelene chlorotrifluoroethylene (ECTFE), polytetrafluoroethylene (PTFE), fluorinated ethylene propylene copolymer (FEP), nylon, Pebax® resin, Vestamid® nylon, Tecoflex® resin, Halar® resin, Hyflon® resin, Pellathane® resin, combinations thereof, and the like. Catheter **20** includes an aperture formed at the distal rounded end **24** allowing advancement over a guidewire **26.**

Balloon **30** can be any variety of balloons or other devices capable of expansion (i.e., by filling with a fluid). Balloon **30** can be manufactured from any sufficiently elastic material such as polyethylene, polyethylene terephthalate (PET), nylon, or the like. Those skilled in the art will recognize that the microdarts **40** can be expanded using a variety of means and that the present invention is not limited to balloon expansion.

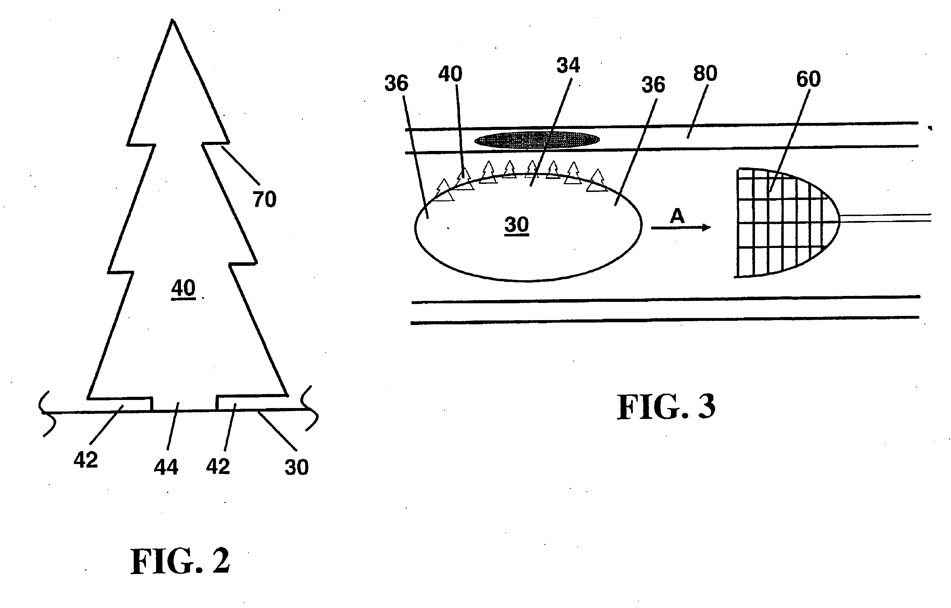

Referring to **FIG. 2****,** a microdart **40** is shown operably attached to the balloon **30** surface. In one embodiment, the microdart **40** includes barbs **70.** In one embodiment, barbs 70 comprise one or more angled surfaces for grabbing tissue and to prevent removal following tissue insertion. Specifically, the microdart **40** includes a shape that allows insertion into tissue with substantially less force than that which is required for removal. In this case, the microdart **40** is shaped similar to a pine tree. In another embodiment, barbs 70 may be shaped like a fish hook barb. In one embodiment, microdart **40** is long enough to ensure penetration into the vessel wall, but short enough to prevent the risk of plaque rupture and to reduce the disturbance of blood flow patterns following encapsulation.

In one embodiment, the microdart **40** includes at least one, in this case two, perforations **42** flanking a base portion **44.** Perforations facilitate removal (e.g., "breaking off") of the microdart **40** from the balloon **30** once the microdart **40** has been inserted into a vessel wall. In one embodiment, the microdart **40** is manufactured substantially from a biodegradable material. Examples of biodegradable materials include, but are not limited to, polylactide-co-glycolide (PLGA), polyglycolyic acid (PGA), poly-lactic acid (PLA), polydioxanone, poly-caprolactone, collagen, polyorthoesters, polyanhydrides, magnesium-based alloys, hydroxyapetite, copolymers and combinations thereof. Those skilled in the art will recognize that the shape and constituent material of the microdarts can vary from the described and illustrated embodiments. In one embodiment, the microdarts are composed of a combination of biodegradable and biostable materials. In another embodiment, the microdarts are composed of biostable material. The microdarts may be composed of biostable materials such as, for example, nitinol, stainless steel, and cobalt-chromium based alloys.

In one embodiment, the microdarts **40** are variably sized. For example, as shown in **FIG. 3****,** the balloon **30** can be generally oval. As such, the central portion **34** of the balloon **30** (when inflated) contacts a vessel wall **80** to a greater extent than does the balloon's **30** end portions **36.** To allow the microdarts **40** positioned adjacent the end portions **36** to penetrate the vessel wall **80** at least as deeply as those in the central portion **34,** the end portion **36** microdarts **40** can be sized substantially longer than the microdart disposed at or near the central portion of the balloon. As another example, the microdarts **40** are differentially sized/shaped to increase or decrease the degree of penetration within the vessel. In another embodiment, the balloon **30** is cylindrical. In this embodiment, the microdarts **40** are substantially the same size. As such, the microdarts **40** can penetrate the vessel wall **80** at substantially the same depth.

In one embodiment, an emboli protection device **60** is positioned downstream from the balloon **30.** The direction of blood flow is represented by arrow A. Device **60** is pre-positioned before the balloon **30** is inflated in the event that the vulnerable plaque ruptures during treatment. As such, any loose emboli and cellular debris are captured thereby reducing the potential consequences of, for example, stroke and heart-attack.

In one embodiment, the microdarts **40** are differentially distributed, concentrated, or oriented on the balloon **30** to correspond to the vulnerable plaque or lesion on the vessel wall. In one embodiment, the microdarts **40** are concentrated on a single region of the balloon **30** to correspond to the vulnerable plaque that is localized on a single vessel wall side.

In one embodiment, the microdarts **40** include at least one therapeutic agent. The therapeutic agent is one or more drugs, polymers, a component thereof, a combination thereof, and the like. For example, the therapeutic agent can include a mixture of a drug and a polymer as known in the art. Some exemplary drug classes that can be included are antiangiogenesis agents, antiendothelin agents, antimitogenic factors, antioxidants, antiplatelet agents, antiproliferative agents, antisense oligonucleotides, antithrombogenic agents, calcium channel blockers, clot dissolving enzymes, growth factors, growth factor inhibitors, nitrates, nitric oxide releasing agents, vasodilators, virus-mediated gene transfer agents, agents having a desirable therapeutic application, and the like. Specific example of drugs include abciximab, angiopeptin, colchicine, eptifibatide, heparin, hirudin, lovastatin, methotrexate, streptokinase, taxol, ticlopidine, tissue plasminogen activator, trapidil, urokinase, and growth factors VEGF, TGF-beta, IGF, PDGF, and FGF.

The polymer generally provides a matrix for incorporating the drug within the coating, or can provide means for slowing the elution of an underlying therapeutic agent when it comprises a cap coat. Some exemplary biodegradable polymers that can be adapted for use with the present invention include, but are not limited to, polycaprolactone, polylactide, polyglycolide, polyorthoesters, polyanhydrides, poly(amides), poly(alkyl-2-cyanocrylates), poly(dihydropyrans), poly(acetals), poly(phosphazenes), poly(dioxinones), trimethylene carbonate, polyhydroxybutyrate, polyhydroxyvalerate, their copolymers, blends, and copolymers blends, combinations thereof, and the like.

Solvents are typically used to dissolve the therapeutic agent and polymer to comprise a therapeutic agent coating solution. Some exemplary solvents that can be adapted for use with the present invention include, but are not limited to, acetone, ethyl acetate, tetrahydrofuran (THF), chloroform, N-methylpyrrolidone (NMP), methylene chloride, and the like.

Those skilled in the art will recognize that the nature of the drug and polymer can vary greatly and are typically formulated to achieve a given therapeutic effect, such as limiting restenosis, thrombus formation, hyperplasia, etc. Once formulated, in one embodiment, a therapeutic agent solution (mixture) comprising the coating can be applied to the microdarts **40** by any of numerous strategies known in the art including, but not limited to, spraying, dipping, rolling, nozzle injection, and the like. Numerous strategies of applying the coating in accordance with the present invention are known in the art. In another embodiment, the therapeutic agent can be integrated throughout the material of the microdart **40.** In yet another embodiment, the therapeutic agent can be administered via a conduit within the catheter **20** and through an aperture (not shown) adjacent the balloon **30** or, alternatively, a drug reservoir within or adjacent the balloon **30.**

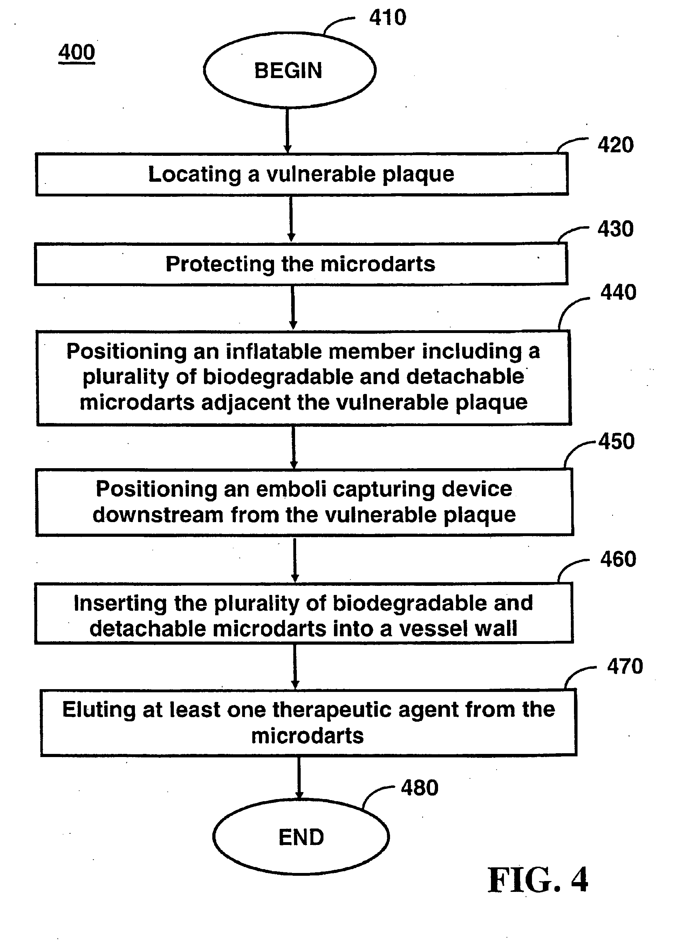

**FIG. 4** illustrates a flowchart of a method 400 of treating a vascular condition. In one example, the treatment of a vascular condition comprises treating a vessel wall that includes a vulnerable plaque lesion. The method **400** begins at step **410.**

At step **420,** a vulnerable plaque is located. Numerous methods are known in the art for locating vulnerable plaque. Examples include, but are not limited to, devices that detect localized changes in temperature, pH, and/or inflammation. Although it is desirable to specifically locate the vulnerable plaque, the present invention is not limited to having located the vulnerable plaque. Those skilled in the art will appreciate that the present invention may be used even if a certain region of vasculature is merely suspected of having vulnerable plaque or other vascular anomaly.

At step **430,** the microdarts **40** are protected. In one example, a sheath **41** is disposed over the balloon **30** to prevent contact between the microdarts **40** and the vessel walls. In another embodiment, a hydrogel coating is used to cover the microdarts **40** to provide protection as well as lubrication during catheter **20** advancement.

At step **440**, the balloon **30** is positioned adjacent the vulnerable plaque. In one example, the catheter **20** is advanced to the treatment site including the vulnerable plaque over a pre-positioned guidewire **26.** In one example, at least one radiopaque marker can be disposed on the balloon **30,** catheter **20,** and or component thereof to allow *in situ* visualization and proper advancement, positioning, and deployment of the microdarts **40.** The marker(s) can be manufactured from a number of materials used for visualization in the art including radiopaque materials such as platinum, gold, tungsten, metal, metal alloy, and the like. Marker(s) can be visualized by fluoroscopy, IVUS, and other methods known in the art. Those skilled in the art will recognize that numerous devices and methodologies may be utilized for positioning an intraluminal stent.

In one example of method 400, a distal protection device **60** is positioned downstream from the vulnerable plaque to protect against accidental vulnerable plaque rupture, step **450.** Those skilled in the art will recognize that emboli may not necessarily be released during treatment of the vulnerable plaque. Rather, the distal protection device **60** is used as a protective measure to capture any emboli that may result due to the rupture of the vulnerable plaque.

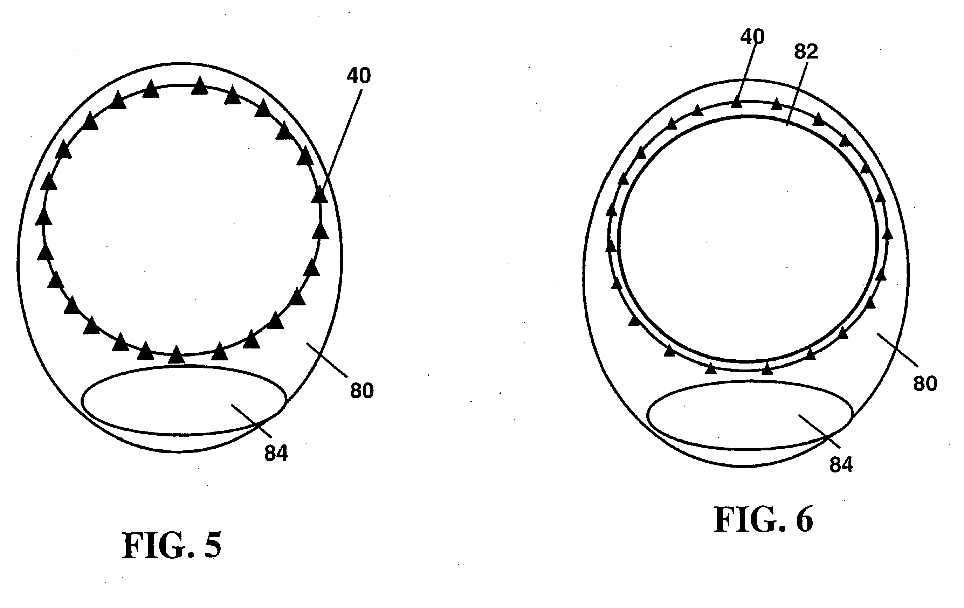

At step **460,** the microdarts **40** are inserted into a vessel wall. In one example, the balloon **30** is expanded axially into contact with the vessel wall via an inflation lumen. If a sheath **41** is present, it is retracted prior to inflation of the balloon **30.** In another embodiment, the microdarts **40** can be designed to puncture through the sheath **41** thereby not requiring retraction of the sheath **41.** As the balloon inflates **40,** the microdarts **40** are inserted into the vessel wall. Preferably, the microdarts **40** do not compromise the integrity of the fibrous cap thereby leaving the vulnerable plaque intact. After the balloon **30** has been inflated and the microdarts **40** inserted, the microdarts **40** are removed from the balloon **30,** but remain in the vessel wall. To achieve this, in one example, the catheter **20** is twisted slightly. The torque exerted on the balloon **30** breaks the microdarts **40** off the balloon **30** at the base portion **44.** The perforations **42** reduce the force needed to break the microdarts **40** off the balloon **30** surface. Balloon **30** can then be deflated and removed along with the catheter **20** and guidewire **26** leaving the microdarts **40** behind in the vessel wall **80** as shown in **FIG. 5****.**

At step **470,** at least one therapeutic agent is eluted from the microdarts **40.** In one example, the microdarts **40** degrade over time as they are manufactured from a biodegradable material. The rate of microdart **40** degradation can vary based on the size, constituent material, and other factors related to the microdart material. The microdarts **40** are encapsulated after insertion thereby stabilizing the vessel wall **80** and reducing the possibility of vulnerable plaque **84** rupture, cracking, and bleeding. The stabilization can occur via the development of a thin fibrous cap **82** that results from the biological reaction to the microdarts **40,** as shown in **FIG. 6****.** One skilled in the art will realize that the stabilization of the fibrous cap could also be achieved using microdarts fabricated from non-degradable, biostable materials. It should also be appreciated that the system of the present invention can also be applied to prevent the rupture of other thin walled biologic structures such as, for example, aneurysmal sacs.

At step **480,** the method terminates and can be repeated as necessary.

While the embodiments of the invention disclosed herein are presently considered to be preferred, various changes and modifications can be made without departing from the spirit and scope of the invention. For example, the catheter, inflatable member, and microdarts are not limited to the illustrated and described embodiments. In addition, the examplary method disclosed for treating a vascular condition may vary. For example, additional steps may be performed in addition to those described.

Upon reading the specification and reviewing the drawings hereof, it will become immediately obvious to those skilled in the art that myriad other embodiments of the present invention are possible, and that such embodiments are contemplated and fall within the scope of the presently claimed invention. The scope of the invention is indicated in the appended claims, and all changes that come within the meaning and range of equivalents are intended to be embraced therein.

## Claims

1. A system (10) for treating a vascular condition, the system comprising:
a catheter (20);
an inflatable member (30) operably attached to the catheter (20); and
a plurality of microdarts (40) disposed on at least a portion of the inflatable member (30),
**characterized in that** the microdarts (40) are detachable from the inflatable member (30).

2. The system of claim 1 wherein the microdarts (40) include at least one therapeutic agent.

3. The system of claim 1 wherein the microdarts (40) are barbed.

4. The system of claim 1 wherein the microdarts (40) are at least partly comprised of biodegradable materials.

5. The system of claim 1 wherein the microdarts (40) comprise at least one perforation adjacent the inflatable member (30).

6. The system of claim 1 wherein the microdarts (40) comprise a plurality of microdarts having a distribution of sizes, geometries, or combinations thereof.

7. The system of claim 1 wherein the microdarts (40) are differentially distributed on the inflatable member (30).

8. The system of claim 1 further comprising a protective member (41) operably attached to the inflatable member (30).

9. The system of claim 1 further comprising an emboli protection device (60) positioned downstream from the inflatable member (30).

10. The system according to any of the preceding claims, the system further comprising:
means for locating a vulnerable plaque;
means for inserting the plurality of detachable microdarts (40) into a vessel wall.

11. The system of claim 10 further comprising means for protecting the microdarts (40).

12. The system of claim 10 further comprising means for capturing emboli downstream from the vulnerable plaque.

## Patentansprüche

1. System (10) zur Behandlung eines Gefäßleidens, wobei das System das Folgende umfasst:
einen Katheter (20);
ein aufblasbares Element (30), welches für den Betrieb mit dem Katheter (20) verbunden ist; und
mehrere Mikrodornen (40), welche zumindest an einem Abschnitt des aufblasbaren Elements (30) angeordnet sind,
**dadurch gekennzeichnet, dass** die Mikrodornen (40) von dem aufblasbaren Element (30) ablösbar sind.

2. System nach Anspruch 1, wobei die Mikrodornen (40) mindestens ein Therapeutikum umfassen.

3. System nach Anspruch 1, wobei die Mikrodornen (40) Widerhaken aufweisen.

4. System nach Anspruch 1, wobei die Mikrodornen (40) zumindest zum Teil aus biologisch abbaubaren Materialien bestehen.

5. System nach Anspruch 1, wobei die Mikrodornen (40) mindestens eine Perforation in Nachbarschaft zu dem aufblasbaren Element (30) aufweisen.

6. System nach Anspruch 1, wobei die Mikrodornen (40) mehrere Mikrodornen umfassen, welche eine Größenverteilung, eine Geometrieverteilung oder Kombinationen daraus aufweisen.

7. System nach Anspruch 1, wobei die Mikrodornen (40) auf dem aufblasbaren Element (30) unterschiedlich verteilt sind.

8. System nach Anspruch 1, welches ferner ein Schutzelement (41) umfasst, welches für den Betrieb mit dem aufblasbaren Element (30) verbunden ist.

9. System nach Anspruch 1, welches ferner eine Gefäßpfropfen-Schutzvorrichtung (60) umfasst, welche stromabwärts des aufblasbaren Elements (30) angeordnet ist.

10. System nach einem der vorhergehenden Ansprüche, welches ferner das Folgende umfasst:
Mittel zum Lokalisieren einer vulnerablen Plaque;
Mittel zum Einführen der mehreren ablösbaren Mikrodornen (40) in eine Gefäßwand.

11. System nach Anspruch 10, welches ferner Mittel zum Schutz der Mikrodornen (40) umfasst.

12. System nach Anspruch 10, welches ferner Mittel zum Einfangen von Gefäßpfropfen stromabwärts der vulnerablen Plaque umfasst.

## Revendications

1. Système (10) pour le traitement d'une condition vasculaire, le système comprenant :
un cathéter (20) ;
un élément gonflable (30) fonctionnellement relié au cathéter (20) ; et
une pluralité de micro-dards (40) disposés sur au moins une portion de l'élément gonflable (30),
**caractérisé en ce que** les micro-dards (40) sont détachables de l'élément gonflable (30).

2. Système selon la revendication 1, dans lequel les micro-dards (40) comprennent au moins un agent thérapeutique.

3. Système selon la revendication 1, dans lequel les micro-dards (40) sont barbelés.

4. Système selon la revendication 1, dans lequel les micro-dards (40) sont au moins partiellement constitués de matériaux biodégradables.

5. Système selon la revendication 1, dans lequel les micro-dards (40) comprennent au moins une perforation adjacente à l'élément gonflable (30).

6. Système selon la revendication 1, dans lequel les micro-dards (40) comprennent une pluralité de micro-dards avec une variété de tailles, de géométries ou de combinaisons de celles-ci.

7. Système selon la revendication 1, dans lequel les micro-dards (40) sont répartis de façon différentielle sur l'élément gonflable (30).

8. Système selon la revendication 1, comprenant en outre un élément protecteur (41) fonctionnellement relié à l'élément gonflable (30).

9. Système selon la revendication 1, comprenant en outre un dispositif anti-embolie (60), positionné en aval de l'élément gonflable (30).

10. Système selon l'une quelconque des revendications précédentes, le système comprenant en outre :
un moyen pour la localisation d'une plaque vulnérable ;
un moyen pour l'insertion de la pluralité de micro-dards détachables (40) dans une paroi de vaisseau.

11. Système selon la revendication 10, comprenant en outre un moyen pour la protection des micro-dards (40).

12. Système selon la revendication 10, comprenant en outre un moyen pour la capture de l'embolie en aval de la plaque vulnérable.
